# EUROPÄISCHE PATENTSCHRIFT

(11) **EP 1 337 349 B1**
(45) Veröffentlichungstag und Bekanntmachung des Hinweises auf die Patenterteilung: **09.03.2011**
(21) Anmeldenummer: 01985344.9
(22) Anmeldetag: 28.11.2001
(51) Int. Cl.: B05C 11/10, A61F 13/15, A61F 13/02, B05B 12/08, B05B 12/12, G05D 7/06

(54) **VORRICHTUNG ZUM GEREGELTEN AUFTRAGEN VON KLEB- UND/ODER DICHTSTOFFEN**
DEVICE FOR REGULATED APPLICATION OF ADHESIVES AND/OR SEALANTS
DISPOSITIF D'APPLICATION REGULEE D'AGENTS ADHESIFS ET/OU D'ETANCHEIFICATION

(30) Priorität: 01.12.2000 DE 10060030; 25.08.2001 DE 10141676
(43) Veröffentlichungstag der Anmeldung: 27.08.2003
(73) Patentinhaber: Henkel AG & Co. KGaA, 40589 Düsseldorf (DE); Produktionstechnik Industrieservice Borst, 63694 Limeshain (DE)
(72) Erfinder: HOFFMANN, Gunter, 73441 Bopfingen (DE); KELS, Volker, 41470 Neuss (DE); HURDELBRINK, Jörg, 06896 Reinsdorf (DE); SCHOLTA, Richard, 51069 Köln (DE); BORST, Willi, 61197 Florstadt (DE); HEUME, Roland, 63688 Gedern (DE)
(86) Internationale Anmeldenummer: PCT/EP2001/013924
(87) Internationale Veröffentlichungsnummer: WO 2002/043878

(56) Entgegenhaltungen:
- EP-A- 0 993 873
- DE-U- 20 100 107
- DE-U- 29 620 763
- DE-U1- 9 101 070
- US-A- 3 887 110
- US-A- 5 054 650

## Beschreibung

Die Erfindung betrifft eine Vorrichtung zum geregelten Auftragen von Kleb- und/oder Dichtstoffen auf Trägermaterialien. Desweiteren betrifft die Erfindung die Verwendung dieser Vorrichtung sowie ein Verfahren unter Verwendung dieser Vorrichtung zum geregelten Auftragen von Kleb- und/oder Dichtstoffen auf Trägermaterialien.

In vielen Fällen müssen beispielsweise kontinuierliche Kleb- und/oder Dichtstoffbänder auf ein Trägermaterial aufgetragen werden, welches in Endlosform (z. B. Materialbahnen) oder in Form von einander unmittelbar folgenden Materialabschnitten respektive Materialzuschnitten an einer Vorrichtung, prinzipiell bestehend aus einem Auftragskopf mit Auftragsdüse, vorbeigeführt werden. In anderen Fällen besteht das Bedürfnis, mehrere Auftragstellen desselben Trägermaterials mit dem gleichen Kleb- und/oder Dichtstoff zu versehen, wobei je nach Fall entweder kontinuierliche Kleb- und/oder Dichtstoffbänder, Kleb- und/oder Dichtstoffpunkte, in der Länge begrenzte Kleb- und/oder Dichtstoffraupen oder Kleb- und/oder Dichtstoffflächen zu erzeugen sind. Beispielsweise müssen bei der Herstellung von Packungen Materialbahnen oder Zuschnitten zur Fixierung in den die Packungen bildenden Positionen mit Klebstoff versehen werden, in Form sogenannter Leimpunkte, Leimraupen oder Leimflächen.

Für die Dosierung der Kleb- und/oder Dichtstoffe weisen die Auftragsköpfe in der Regel Ventile auf, die die Zufuhr der Kleb- und/oder Dichtstoffe zur Auftragsdüse über elektronische Steuereinheiten regeln.

Für die überwiegende Zahl der Anwendungen ist es oft wünschenswert, ein gleichmäßiges Auftragsmuster und eine definierte Auftragsmenge bzw. Dosierung des aufzutragenden Kleb- und/oder Dichtstoffes zu erzielen und insbesondere die Erzielung der gewünschten Auftragsmenge zu kontrollieren.

Eine Kontrolle des Auftragens des Kleb- und/oder Dichtstoffes ist erforderlich, da beispielsweise eine Verstopfung im Leitungssystem, des Auftragskopfes, der Auftragsdüse, in eventuell vorhandenen Filtern oder eine Undichtigkeit an einer beliebigen Stelle der Vorrichtung nicht immer mit Sicherheit auszuschliessen ist. Differenzen zwischen der gewünschten und der praktisch erzielten Auftragsmenge können beispielsweise durch fertigungsbedingte Schwankungen des Förderpumpendruckes oder durch Viskositätsänderungen, beispielsweise hervorgerufen durch Temperaturänderungen, auftreten. Solche Schwankungen können insbesondere dazu führen, daß die aufgetragene Kleb- und/oder Dichtstoffmenge für die erforderliche Verklebung und/oder Abdichtung des Trägermaterials zu gering ist. Solchen Effekten versucht man daher häufig entgegenzuwirken, indem zu der theoretisch erforderlichen Auftragsmenge noch eine Sicherheitsreserve des aufzutragenden Kleb- und/oder Dichtstoffes berücksichtigt wird.

Sicherheitsreserven werden insbesondere in Verfahren berücksichtigt, bei denen die Trägermaterialien schwierig zu verklebende oder abzudichtende Oberflächen besitzen, beispielsweise sind diese Oberflächen lackiert, bedruckt oder oberflächenkaschiert. Die Berücksichtigung einer Sicherheitsreserve ist auch dann erforderlich, wenn eine hohe Zugbeanspruchung auf der Verklebung lastet. Dies ist beispielsweise der Fall, wenn Trägermaterialien unter erzwungener Formgebung miteinander verklebt werden und diese Trägermaterialien das Bestreben zeigen, wieder in ihre Ausgangsform zurückzukehren. Beispielsweise geschieht dieses, wenn Trägermaterialien über Kanten verklebt werden, z. B. bei der Kantenverleimung von Hölzern. Desweiteren erfolgt die Berücksichtigung der Sicherheitsreserve in Verfahren, bei denen eine hohe Wärmestandfestigkeit des Dicht- oder Klebstoffes gefordert wird, beispielsweise beim Befüllen von verklebten oder abgedichteten Trägermaterialien mit heißem Füllgut. Als Wärmestandfestigkeit wird hier allgemein das Vermögen einer Klebschicht definiert, einer Temperaturbeanspruchung gegenüber langzeitig ohne Deformation zu widerstehen.

Die Kontrolle, ob der gewünschte Auftrag des Kleb- und oder Dichtstoffes auf dem Trägermaterial vorhanden ist, erfolgt üblicherweise stichprobenartig oder kontinuierlich, beispielsweise über Sichtkontrollen und/oder ergänzend
a) Feuchtigkeitsmessungen, wenn der aufgetragene Klebstoff Wasser- oder Lösemittel-basierend ist,
b) Infrarotsensoren, um über die abgestrahlte Wärme die Dosierung des aufgetragenen Kleb- und/oder Dichtstoffes auf dem Trägermaterial zu bestimmen,
c) gravimetrisch, indem vor und nach dem Auftrag des Kleb- und/oder Dichtstoffes entsprechend markierte Trägermaterialien gewogen werden.
d) Mikrowellen- bzw. Ultraschallwellentechnik zur Messung von Kleb- und/oder Dichtstoffspuren oder Kleb- und/oder Dichtstoffschichten.

Die Genauigkeit der gelisteten Kontrollmöglichkeiten ist, bedingt durch die Meßmethoden und durch zusätzliche äußere Einflußfaktoren (z. B. Luftfeuchtigkeit), nicht sehr hoch. Dieses ist ein weiterer Grund für die üblicherweise praktizierte Zusatzdosierung des aufzutragenden Kleb- und/oder Dichtstoffes als Sicherheitsreserve.

Ist der Auftrag des Kleb- und/oder Dichtstoffes auf das Trägermaterial gar nicht oder nur unzureichend erfolgt, so hat dies im allgemeinen den Ausschuß des Trägermaterials zur Folge. Wird ferner berücksichtigt, daß heutzutage viele Verfahren, in denen die Kleb- und/oder Dichtstoffe auf Trägermaterialien aufgetragen werden, mit hohen Geschwindigkeiten ablaufen, ist der wirtschaftliche Verlust durch Ausschuß und dem damit verbundenen Maschinenstillstand, bedingt durch Reinigungs- und Reperaturarbeiten, nicht unerheblich.

Um auf Störungen des Auftrags der Kleb- und/oder Dichtstoffe auf Trägermaterialien schneller reagieren zu können, sind daher Verfahren vorteilhaft, bei denen eine Kontrolle des Auftrags des Kleb- und/oder Dichtstoffes auf der Strecke zwischen dem Vorratsbehälter für den Kleb- und/oder Dichtsstoff und der Auftragsdüse und nicht erst ausschließlich auf dem Trägermaterial erfolgt.

### Solche Verfahren sind bekannt.

In der EP 0887 721 A1 wird ein monitoring system offenbart, in dem ein Monitor periodisch Eingangssignale eine Sensors sammelt und diese gesammelten Signale mit gespeicherten Alarm-Limit-Werten vergleicht. Der Sensor erfasst hierbei ein Charakteristikum eines Flüssigkeitsstroms durch einen Verteiler, beispielsweise den Flüssigkeitsdüsendruck. Ein im Auftragskopf integrierter Druckaufnehmer mißt hierbei den statischen Druck der Flüssigkeit bei geschlossener Düse sowie den dynamischen Druck der Flüssigkeit bei geöffneter Düse. Bei geöffneter Düse fällt der Druck in Abhängigkeit von der Durchflußmenge ab. Die gemessenen Drücke werden mit vorher festgelegten Referenzdrücken verglichen und somit überwacht. Dieses Verfahren läßt Rückschlüsse zu auf richtigen Pumpendruck sowie auf die Durchflußmenge im Auftragskopf. Je geringer allerdings der Durchfluß des Flüssigkeitsstroms in der Düse ist, desto geringer ist der gemessene Differenzdruck zwischen dem dynamischen Druck und dem Referenzdruck. Es liegt daher nahe, daß beim Durchfluß von Kleinstmengen an Flüssigkeit, beispielsweise weniger als 100 mg Flüssigkeit, kein ausreichender Differenzdruck messbar ist.

In der Gebrauchsmusterschrift DE 296 20 763 U wird ein Gerät zum Auftragen von Klebstoff beschrieben, bestehend aus einem Klebstoffvorratsbehälter, einer Förderpumpe und einem Auftragskopf mit mindestens einer Auftragsdüse, wobei der Klebstoffvorratsbehälter, die Förderpumpe und der Auftragskopf durch eine den Klebstoff führende Leitung verbunden sind, und welches zwischen der Förderpumpe und der mindestens einen Auftragsdüse mindestens einen Klebstoffvolumenstromsensor angeordnet enthält. Dieser Klebstoffvolumenstromsensor dient dazu, den tatsächlich von der Förderpumpe zur mindestens einen Auftragsdüse geförderten Klebstoffvolumenstrom zu messen. Sollte der Sensor während eines Arbeitstaktes feststellen, daß kein oder nicht ausreichend Klebstoff gefördert wird, so bewirkt eine entsprechende Überwachungsschaltung eine sofortige und für das Bedienpersonal ohne weiteres erkennbare Unterbrechung des Produktionsablaufes.

Die beschriebenen Vorrichtungen kontrollieren den Auftrag von Klebstoffen und lösen einen Alarm bei einer Störung des Auftrags aus, werden aber nicht zur Regelung einer präzisen Dosierung von Klebstoffen benutzt. Ferner ist mit diesen Vorrichtungen weder eine Mengenbestimmung noch eine Dichtebestimmung der Flüssigkeit durchführbar.

Das Gebrauchsmuster DE-U 91 01 070 beschreibt eine Vorrichtung zum strangförmigen Auftragen von fließfähigen Massen auf einen Träger. Dabei wird das Material über eine Düse und eine Dosiereinrichtung zugeführt, wobei über einen Regelkreis die Volumenmenge pro Wegeinheit der aufzutragenden Masse geregelt wird. Dabei soll das Messglied weit vom Austrittspunkt der Auftragsmasse entfernt angeordnet sein.

Das Gebrauchsmuster DE-U 91 01 070 beschreibt eine Vorrichtung zum strangförmigen Auftragen von fließfähigen Massen auf einen Träger. Dabei wird das Material über eine Düse und eine Dosiereinrichtung zugeführt, wobei über einen Regelkreis die Volumenmenge pro Wegeinheit der aufzutragenden Masse geregelt wird. Dabei soll das Messglied weit vom Austrittspunkt der Auftragsmasse entfernt angeordnet sein.

Die US 5,646,737 offenbart eine Vorrichtung zum Beschichten mit flüssigen, bevorzugt fotografischen Materialien. Mit dieser Vorrichtung ist eine Schichtvermessung der aufgetragenen Flüssigkeit möglich, wobei aus dem Schichtprofil in x- und y-Richtung die Auftragsmenge errechnet wird. In Verbindung mit einem Encoder ist man in der Lage eine Auftragsmengensteuerung durchzuführen. Die Schichtvermessung setzt allerdings voraus, dass das beschichtete Material absolut eben ist, die Materialstärke absolut konstant bleibt und die Beschichtung kontinuierlich erfolgt. Entsprechend ist diese Auftrags- und Messvorrichtung nur für die Flächenbeschichtung auf Folien bzw. folienähnlichen Materialien anwendbar.

Aufgabe der vorliegenden Erfindung war es, die Nachteile des Standes der Technik zu vermeiden und bestehende Verfahren und Vorrichtungen zum Auftragen von Kleb- und Dichtstoffen zu verbessern und zu vereinfachen. Hierzu gehörte insbesondere die Aufgabe, eine verbesserte Kontrolle des Auftrags, eine genauere Messung der aufgetragenen Menge sowie eine Regelung der Auftragsmenge von Kleb- und Dichtstoffen, unabhängig von dem Trägermaterial, der Materialoberfläche (z. B. Folien, Vlies, Karton, Holz, etc.) sowie der Auftragsform (z. B. Flächenbeschichtung, Spiralsprühauftrag, Punktauftrag, Raupenauftrag), zu ermöglichen. Eine genaue Messung der aufgetragenen Menge soll sowohl bei intermittierender als auch bei kontinuierlicher Auftragsform durchführbar sein.

Die erfindungsgemäße Lösung der Aufgabe ist den Ansprüchen zu entnehmen.

In den Ausführungsbeispielen werden die Kleb- oder Dichtstoffe ausgehend von einem Vorratsbehälter (1), mit der Förderpumpe (2) über ein Leitungssystem (7), welches starre und/oder flexible Leitungen besitzt und gekühlt oder beheizt werden kann, zu einem Auftragskopf (3) gefördert und dort mit Hilfe der mindestens einen Auftragsdüse (4) auf ein Trägermaterial (11) aufgegeben, welches sich auf einer kontinuierlich oder diskontinuierlich laufenden Transporteinrichtung (12), beispielsweise einem Förderband, befindet.

Zur Druckerzeugung in dem Leitungssystem (7) können intermittierend arbeitende Förderpumpen (2), wie z. B. Kolbenpumpen, oder kontinuierlich arbeitenden Förderpumpen (2), wie z. B. Zahnradpumpen, eingesetzt werden. Die Förderpumpe (2) kann sowohl pneumatisch als auch elektronisch betrieben werden.

Der Volumenstrom des geförderten Kleb- und/oder Dichtstoffes wird mittels eines Volumenstromsensors (5) gemessen.

Erfindungsgemäß ist der Volumenstromsensor (5) Bestandteil des Auftragkopfes (3) und, in Flußrichtung des geförderten Kleb- und/oder Dichtstoffes gesehen, unmittelbar vor der mindestens einen Auftragsdüse (4) angeordnet.

Dadurch können Meßverzögerungen und der Einfluß von Druckschwankungen auf das Meßsystem weitgehend ausgeschlossen werden.

Nach dem Prinzip einer Zellradschleuse wird die geförderte Menge des durch den Volumenstromsensor (5) hindurch strömenden Kleb- und/oder Dichtstoffes erfaßt und in Form von elektrischen Impulsen an die Kontroll-Einheit (6) gesendet.

Der Volumenstromsensor (5) kann als Bestandteil eines Regelkreises gesehen werden, der den tatsächlich geförderten Volumenstrom der (des) Kleb- und/oder Dichtstoffe(s) kontrolliert und auf Änderungen, beispielsweise einen Mangel an Kleb- und/oder Dichtstoff, aufmerksam macht.

Erfindungsgemäß ist der Volumenstromsensor (5) in Form einer umgekehrt arbeitenden Zahnradpumpe ausgebildet ist, da diese Art der Volumenstrommessung besonders kleine Massentransporte bis zu einer Untergrenze von 10 mg, bezogen auf eine Dichte von 1 g/ml, differenziert auflösen kann.

Der Volumenstromsensor (5) mißt jeweils durch die Verzahnung vorgegebene Teilmengen des Kleb- und/oder Dichtstoffes. Für jede Teilmenge, die durch den Volumenstrom hervorgerufene Drehbewegung der ineinander kämmenden Zähne gefördert wird, wird über den magneto-elektrischen Sensor ein elektrischer Impuls abgegeben. In der Regel handelt es sich bei dem magneto-elektrischen Sensor um einen Differenzialfeldplattenfühler, der über dem Zahnkranz eines der beiden Meßwerkzahnräder liegt und der für jeden unter ihm durchlaufenden Zahn einen elektrischen Impuls abgibt. Der mechanische Teil eines derartigen Sensors ist beispielsweise nach der DE 40 40 409 C1 bekannt, während bezüglich des zugehörigen, eigentlichen Meßsensors auf die DE 40 42 397 C2 verwiesen wird. Zur Erzielung einer höheren Meßgenauigkeit können auch mehrere Differentialfeldplattenfühler verwendet werden. In einer bevorzugten Ausführungsform der Erfindung ist der Volumenstromsensor mit zwei Differentialfeldplattenfühlern mit jeweils getrenntem Vorverstärker ausgestattet. Die Impulse auf den entsprechenden Kanälen 1 und 2 sind um 90° versetzt und müssen als symmetrische Phasen erscheinen. Die hierzu benötigte präzise Einbaulage des Differentialfeldplattenfühlers an den Volumenstromsensor zur induktiven Erfassung der Meßzellenräder wird zweckmäßigerweise durch eine hochtemperaturfeste Kunststoffaufnahme, z. B. Teflon, erreicht.

Der Volumenstromsensor (5) eignet sich zum Einsatz in einem Temperaturbereich zwischen 20 °C bis 300 °C, bevorzugt 80 °C bis 250 °C und insbesondere bevorzugt zwischen 160 °C und 210 °C.

Je nach Abstand der Meßwerkzahnräder zueinander liegt die Auflösung zwischen 0,001 ml bis 2 ml, bevorzugt zwischen 0,003 ml und 1,5 ml und insbesondere bevorzugt zwischen 0,005 ml und 1 ml. Die Wahl des einzusetzenden Volumenstromsensors (5) hängt von der Viskosität und der Menge des aufzutragenden Dicht- und/oder Klebstoffes ab.

Die vom magneto-elektrischen Sensor abgegebenen elektrischen Impulse werden über eine entsprechende Impuls-Übertragungsleitung (8) an die Kontroll-Einheit (6) mit dazugehörendem Datenverarbeitungsprogramm geleitet.

Die Kontroll-Einheit (6) mit dazugehörendem Datenverarbeitungsprogramm wird im Folgenden als Meß- und Regeleinheit verstanden, die über einen kontinuierlichen Soll-/Ist-Abgleich In-line die Auftragsmenge des Kleb- und/oder Dichtstoffes auf das Trägermaterial (11) kontrolliert und gegebenenfalls an den Sollwert anpaßt.

Das zur Kontroll-Einheit (6) gehörenden Datenverarbeitungsprogramm basiert auf Standard-Software nach der Norm IEC 1131-3, die Sprachelemente und Programmierregeln für die weltweit verbreiteten Programmiersprachen für speicherprogrammierbare Steuerungen betrifft. Konkret erfüllt beispielsweise die Programmiersoftware STEP 7 aus der SIMATIC-Produktfamilie der Firma Siemens diese Norm. Die Software ist den Belangen der erfindungsgemäßen Vorrichtung angepaßt und verarbeitet die für den kontinuierlichen Soll-/Ist-Abgleich erforderlichen Daten, wobei frei wählbare Ober- und Untergrenzen für die Auftragsmenge des Kleb- und /oder Dichtstoffes als Limitwerte gewählt werden, innerhalb derer eine Anpassung an den Sollwert erfolgen soll.

Läßt beispielsweise die geförderte Menge des Kleb- und/oder Dichtstoffes durch eine beginnende Verstopfung des Leitungssystems (7) langsam nach, so wird die Förderpumpe (2) angesteuert und der Auftrag des Kleb- und/oder Dichtstoffes durch Ausgabe eine Analogsignals geregelt und an den Sollwert anpasst.

Handelt es sich bei der Förderpumpe (2) um eine pneumatisch betriebene Förderpumpe, wird ein entsprechender handelsüblicher elektropneumatischer Regler benötigt. Der elektropneumatische Regler wird vorzugsweise unmittelbar am Druckluft-Eingang der pneumatisch betriebenen Förderpumpe angeschlossen. Handelt es sich bei der Förderpumpe (2) um eine elektronisch betriebene Förderpumpe, ist zur Regelung der Pumpenfrequenz eine entsprechende Reglerkarte notwendig.

In einer besonders bevorzugten Ausführungsform enthält die erfindungsgemäße Vorrichtung einen Encoder (13).

Encoder (13) oder auch Drehwinkelgeber dienen dazu, den Drehwinkel bzw. die Länge und Richtung einer Dreh- bzw. Linearbewegung von bewegten Körpern zu erfassen. Die bewegten Körper weisen hierzu eine Stelle mit magnetischer, optischer oder in sonstiger Weise Codierung auf, werden an einem feststehenden Sensorelement vorbeigeführt, wobei in jeder Stellung der codierten Stelle zum Sensor ein Absolutwert hinsichtlich der Winkelstellung und Richtungszahl (z. B. Drehzahl) ermittelbar ist.

Die wesentlichen Bestandteile von beispielsweise optischen Encodern sind das Emittiersystem, eine Rasterplatte, üblicherweise eine Rasterscheibe oder ein Rastersignal und das Detektorsystem. Das Emittiersystem besteht üblicherweise aus einer Leuchtdiode bzw. Laserdiode. Das von der Laserdiode abgestrahlte Lichtbündel wird von der Rasterplatte moduliert. Diese ist mit dem bewegten Körper verbunden und weist ein periodisches Öffnungsmuster auf. Das Detektorsystem erfasst das von der Rasterplatte modulierte Sendersignal der Laserdiode und liefert am Ausgang die Information über Zählimpuls und Richtung der Bewegung.

Prinzipiell können alle auf dem Markt gängigen Encoder verwendet werden, bevorzugt werden Encoder, die mit einer Betriebsspannung von 10 Volt bis 30 Volt arbeiten. Weitere charakteristische Daten des Encoders, beispielsweise Impulszahl, Umdrehungszahl, Übersetzungsverhältnis usw. werden bereits bei der Programmierung der Kontrol-Einheit (6) mit dazugehörendem Datenverarbeitungsprogramm berücksichtigt.

Mit Hilfe des Encoders (13) ist es möglich, den Weg und die dazu benötigte Zeit von Trägermaterialien (11) zu erfassen, die an der mindestens einen Auftragsdüse (4) vorbeigeführt werden.

Encoder (13) und Kontroll-Einheit (6) mit dazugehörendem Datenverarbeitungsprogramm sind über eine entsprechende Übertragungsleitung (14) miteinander verbunden.

Die vom Encoder (13) gelieferten Informationen werden von der Kontroll-Einheit (6) mit dazugehörendem Datenverarbeitungsprogramm erfasst, elektronisch verarbeitet und ermöglichen eine geschwindigkeitsabhängige Regelung der Auftragsmenge sowie der Auftragslänge des Kleb- und/oder Dichtstoffes.

Die geschwindigkeitsabhängige Regelung bezieht sich auf die Geschwindigkeit des Trägermaterials (11), mit der es an der Auftragsdüse (4) vorbei transportiert wird.

Da die vom Encoder (13) gelieferten Informationen von der Kontroll-Einheit (6) mit dazugehörendem Datenverarbeitungsprogramm kontinuierlich erfasst und verarbeitet werden, ist neben der geschwindigkeitsabhängigen Regelung gleichzeitig eine permanente Kontrolle der Dosiermenge des Kleb- und/oder Dichtstoffes sowie der Dosierzeit möglich.

Wie bereits an anderer Stelle ausgeführt, gibt der Volumenstromsensor (5) bei Förderung eines bestimmten Volumens einen elektromagnetischen Impuls ab, beispielsweise bei einem Volumen von 10 ml einen Impuls. Die Wahl der Messzelle ist abhängig von der Viskosität der Flüssigkeit, wobei mit steigender Viskosität eine Messzelle mit größerem Volumen pro Impuls gewählt wird. Je größer die Messzelle, desto größer ist aber auch die Messungenauigkeit. Zur Verbesserung der Messgenauigkeit ist insbesondere beim intermittierenden Auftrag und Kleinstmengenmessung der Einsatz eines Encoders besonders bevorzugt. Im Vergleich zur Auflösung der abgegebenen Impulse des Volumenstromsensors (5) ist die Auflösung der abgegebenen Impulse des Encoders (13) weitaus höher. Bezogen auf eine bestimmte Auftragslänge wird nur ein Impuls des Volumenstromsensors (5), aber eine vielfach höhere Anzahl von Impulsen des Encoders (13) erzeugt. Die unbekannte Menge aufgetragenen Klebstoffes zwischen zwei Impulsen des Volumenstromsensors (5) für eine bestimmte Auftragsmenge wird durch Hinzuziehen eines Umrechnungsfaktors der Impulse Volumenstromsensor pro Impulse Encoder mittels der Kontroll-Einheit (6) mit dazugehörendem Datenverarbeitungsprogramm berechnet.

Die Kontroll-Einheit (6) mit dazugehörendem Datenverarbeitungsprogramm ist so ausgestaltet, dass vor dem Messzyklus die Messung auf Massebestimmung kalibriert ist und somit die tatsächlich aufgetragene Menge des Klebstoffes bestimmt wird.

Die erfindungsgemäße Vorrichtung findet Verwendung in Verfahren zum geregelten Auftragen von Kleb- und/oder Dichtstoffe auf Trägermaterialien (11) nach Anspruch 7.

Bevorzugt wird die Über- oder Unterschreitung der gewählten Limitwerte von der Kontroll-Einheit (6) mit dazugehörendem Datenverarbeitungsprogramm als sogenannte Störung registriert, verarbeitet und zweckmäßigerweise über elektronische und/oder akustische Funktionen als Störungsmeldung signalisiert.

Hierzu verfügt die Kontroll-Einheit (6) mit dazugehörendem Datenverarbeitungsprogramm zweckmäßigerweise über einen Ausgang (10) zur externen Verarbeitung von Störungsmeldungen.

Beispielsweise kann hierüber ein Computermonitor angeschlossen werden und im Falle einer Störung wird diese auf dem Display angezeigt. Zusätzlich oder alternativ können zum Beispiel akustische (z. B. Sirene) oder optische (z. B. Warnlampe) Funktionen über den Ausgang (10) angeschlossen werden. Gegebenenfalls kann die Kontroll-Einheit (6) mit dazugehörendem Datenverarbeitungsprogramm die Förderung des Kleb- und/oder Dichtstoffes aber auch komplett unterbrechen, wenn beispielsweise eine Anpassung an den Sollwert nicht mehr möglich ist und um beispielsweise durch die Störung bedingte notwendige Reinigungsmaßnahmen durchzuführen.

Das Verfahren ist insbesondere zum Auftragen von Leimen, Kleistern, Dispersions-Klebstoffen, Lösungsmittel-Klebstoffen, Kontakt-Klebstoffen, Reaktions-Klebstoffen, Haft-Klebstoffen, Haft-Schmelzklebstoffen und Schmelzklebstoffen geeignet. Als ganz besonders geeignet erweisen sich hierbei Reaktions-Klebstoffe, Haft-Klebstoffe, Haft-Schmelzklebstoffe und Schmelzklebstoffe.

Schmelzklebstoffe sind in der Regel Wasser- und Lösungsmittelfreie Klebstoffe, die auf die zu verklebenden Teile aus der Schmelze aufgetragen werden und nach dem Zusammenfügen beim Abkühlen unter Verfestigung physikalisch abbinden. Schmelzklebstoffe finden eine breite industrielle Anwendung, beispielsweise zum Kleben und Kaschieren in der Möbel-, Schuh-, Elektro-, Verpackungs- und Papierindustrie. In der Verpackungs- und Papierindustrie werden sie beispielsweise zum Versiegeln oder Verschließen von Kartons, zum Lamenieren mehrschichtiger Papiere oder zum Binden von Büchern verwendet.

Zum Herstellen von Medikalartikeln (z. B. Pflaster, Verbandsmaterial) oder Hygieneartikeln (z. B. Windeln, Damenbinden) werden mit Schmelzklebstoffen unterschiedlichste Trägermaterialien miteinander verbunden. So lassen sich Materialien wie Polyolefinfolien, beispielsweise Polyethylenfolien oder Polypropylenfolien, Polyolefinvliese, beispielsweise Polyethylenvliese oder Polypropylenvliese, Polyurethanfolien, Polyurethanschäume, Folien oder Formkörper aus Cellulosederivaten, beispielsweise aus Zellstoff (Tissues), Folien oder Formkörper aus Polyacrylaten oder Polymethacrylaten, Folien oder Formkörper aus Polyester untereinander verbinden. Eine Verbindung ist dabei sowohl unter gleichen Materialien als auch unter verschiedenen Materialien möglich.

Als Etikettierklebstoffe finden Schmelzklebstoffe in der Lebensmittel- und der Getränkeindustrie ein weites Anwendungsfeld. In der Zigarettenindustrie werden Schmelzklebstoffe beispielsweise zum Verkleben des Zigarettenfilters verwendet.

Als Grundstoffe für Schmelzklebstoffe stehen die verschiedenartigsten Polymere/Copolymere zur Verfügung, beispielsweise
- Polykondensate wie Polyamidharze, Copolyamide, Polyamid/EVA-Copolymere, Polyamid/Siloxan-Copolymere, Polyetheramide, Polyesteramidimide, Polyetheresteramide, Polyesteramide und Copolyester, gesättigte Polyester,
- Polyaddukte wie reaktive und nicht reaktive lineare oder leichtverzweigte thermoplastische Polyurethane und
- Polymerisate wie Ethylen-Copolymere, Copolymere von Ethylen mit ungesättigten Carbonsäuren, Ethylen-Vinylacetat-Copolymere, Ethylen-Terpolymere, z.B. Ethylen-Acrylat-Terpolymere, Propylen/Hexen-, SIS- und SBS-Copolymere sowie weitere thermoplastische Elastomere und amorphe Polyolefine, z.B. Polyethylen, über Metallocen-Katalyse hergestellte Polyolefine, insbesondere PP, und schließlich Polybuten.

Bevorzugte Polymere sind Homo- und/oder Copolymere aus olefinischen Monomeren, insbesondere aus alpha-Olefinen. Diese Homo- und/oder Copolymeren werden durch Polymerisation von olefinischen Monomeren mit einer Kettenlänge von C₂ bis C₂₀ hergestellt. Insbesondere sind solche Homo- und/oder Copolymere aus olefinischen Monomeren bevorzugt, die unter Verwendung von Metallocen-Katalysatoren hergestellt wurden. Besonders bevorzugt sind Homo- und/oder Copolymere aus olefinischen Monomeren, die keine funktionellen Gruppen aufweisen.

Die aufgeführten Polymere bestimmen im wesentlichen die eigentlichen Klebschichteigenschaften in Bezug auf Haftung, Festigkeit und Temperaturverhalten. Für die Erzielung weitere spezieller Eigenschaften (z. B. Kohäsion, Viskosität u. a.) dienen Zusätze von beispielsweise klebrigmachenden Harzen, Wachsen, Weichmachern, z. B. Ölen, Stabilisatoren, Antioxidantien und/oder Füllstoffen.

Konkrete Produkte für Verklebungen und/oder Abdichtungen sind beispielsweise von der Firma Henkel KGaA, Dorus oder Teroson erhältlich. Für flexible Verpackungen oder für die Papierveredelung sind es beispielsweise Klebstoffe mit der Bezeichnung Liofol, Lio-Clean oder Liotron. Für die Herstellung von beispielsweise Beschichtungen, Selbstklebeetiketten oder Klebebändern sind Klebstoffe mit der Bezeichnung Euromelt, Dorus-PS, Adhesin J, Sichello J oder Liotron im Einsatz. Bei der Kunststoffetikettierung werden beispielsweise Klebstoffe wie Euromelt, Pekal, Optal oder Smeltan verwendet. Für Heißsiegelbeschichtungen oder Textlamierungen werden beispielsweise Lioseal oder Liotex eingesetzt.

Produkte, wie beispielsweise Technomelt, Adhesin oder Curo kommen im Bereich der Kartonverpackung (Außenkartonverschluß, Faltschachtelverschluss, Kartonaufrichtung), Tiefkühlverpackung oder Palettensicherung zum Einsatz.

Im Hygienebereich (z. B. Babywindeln, Damenhygiene oder Inkontinenz) oder Medikalbereich (z. B. OP-Tücher, Bandagen, Pflaster) werden beispielsweise Sanicare-Klebstoffe eingesetzt. In der Zigarettenfertigung (Seitennahtverschluss, Verpackungen, Filterherstellung usw.) werden beispielsweise Tobacoll-Klebstoffe eingesetzt.

Im Bereich beispielsweise der Isolierglasverklebung, Kabelfüllmassen, Holzverarbeitung oder Schuhfertigung werden beispielsweise Terostat, Macromelt oder Macroplast eingesetzt.

Das Verfahren ist für Kleb- und/oder Dichtstoffe geeignet, die bei den entsprechenden Verarbeitungstemperaturen eine Viskosität von 20 mPas bis 100 000 mPas, bevorzugt 20 mPas bis 40 000 mPas und insbesondere bevorzugt zwischen 20 mPas und 20 000 mPas, gemessen nach ASTM D 3236, aufweisen. Unter entsprechenden Verarbeitungstemperaturen sind Temperaturen zwischen 20 °C bis 300 °C, bevorzugt 40 °C bis 250 °C und insbesondere bevorzugt zwischen 40 °C und 200 °C zu verstehen.

Bevorzugt zeigen diese Kleb- und/oder Dichtstoffe keinen Fadenzug beim Austritt aus der Auftragsdüse (3), insbesondere, wenn kleine Mengen in der Größenordnung von 10 bis 100 mg aufgetragen werden. Im Bereich dieses Kleinstmengenauftrags eignen sich insbesondere Kleb- und/oder Dichtstoffe mit einer Viskosität zwischen 20 mPas und 20 000 mPas, gemessen nach ASTM D 3236, gegebenenfalls in Kombination mit einem Auftragskopf (3) mit mindestens einer Auftragsdüse (4), wobei die mindestens eine Auftragsdüse (4) in Form einer Spiralsprühdüse ausgebildet ist. Idealerweise enthalten die beim Kleinstmengenauftrag aufzutragenden Kleb- und/oder Dichtstoffe keine Feststoffanteile. Sind gegebenenfalls Feststoffanteile vorhanden, so beträgt deren Partikelgröße nicht mehr als 5 Mikrometer.

Insbesondere bevorzugt ist das Verfahren für Kleb- und/oder Dichtstoffe geeignet, die bei der entsprechenden Verarbeitungstemperatur über eine konstante Viskosität mit einer Schwankung von +/- 50 % innerhalb des üblichen Verarbeitungszeitraumes verfügen. Unter dem üblichen Verarbeitungszeitraum sind Zeiten von 15 Minuten bis zu 72 Stunden zu verstehen.

Beispielsweise ist bei Schmelzklebstoffen bekannt, daß sie, abhängig von der Zusammensetzung, unter Temperatureinwirkung stoffliche Veränderungen erfahren können. Dies kann sich, je nach Höhe und Dauer der Temperatureinwirkung, in einem Abdampfen von flüchtigeren Rezepturbestandteilen und/oder in einem zumindest teilweisen thermischen Abbau auswirken. Durch den thermischen Abbau können flüchtige Zersetzungsprodukte entstehen und/oder sogenannte Vercrackungs- oder Verkokungsprodukte, wobei meist eine Vernetzung bereits vorhandener oder durch die Temperatureinwirkung entstehender ungesättigter Verbindungen als Vorstufe der Vercrackung oder Verkokung einsetzt. Diese durch Temperatureinwirkung hervorgerufenen stofflichen Änderungen äußern sich beispielsweise in einer Änderung der Viskosität, aber auch in einer beginnenden oder bewirkten Inhomogenität einer vorher homogenen Zusammensetzung, in einer teilweisen oder vollständigen Unlöslichkeit in meist organischen Lösungsmitteln, in denen die Zusammensetzung vor der Temperatureinwirkung vollständig löslich war und/oder in Form einer Änderung des Farb- und Aggregatzustandes von beispielsweise schwach-gelblichen Flüssigkeiten zu schwarzen, lackartigen Produkten.

Die erfindungsgemäße Vorrichtung eignet sich insbesondere zum geregelten Auftragen und Dosieren von Kleb- und/oder Dichtstoffen im produktionstechnischem Maßstab:
Es können Mengen bis zu 300 l/min, abhängig von der Viskosität des Kleb- und/oder Dichtstoffes, geregelt aufgetragen werden. Die maximale Geschwindigkeit, mit der das Trägermaterial (11) an der Auftragsdüse (4) vorbeigeführt werden kann und noch eine geregelte Auftragung möglich ist, entspricht der Geschwindigkeit der in der Produktion eingesetzten Hochleistungsmaschinen.

Das erfindungsgemäße Verfahren zum geregelten Auftragen von Kleb- und/oder Dichtstoffen auf Trägermaterialien (11) kann sowohl intermittierend als auch kontinuierlich durchgeführt werden.

Je nach Bedarf wird hierzu der Volumenstromsensor (5) in handelsübliche Auftragsköpfe (3) integriert, welche zur Erzeugung von Punkten, Raupen oder zur Flächenbeschichtung mit Kleb- und/oder Dichtstoffen verwendet werden:
- So kann beispielsweise der punktförmige Auftrag von Kleb- und/oder Dichtstoffen ab einer Menge von 10 mg, bezogen auf eine Dichte von 1 g/ml, bis zu 2000 Punkte pro Minute kontrolliert und geregelt werden.
- Durch die vom Encoder (13) an die Kontroll-Einheit (6) mit dazugehörendem Datenverarbeitungsprogramm gelieferten Informationen und deren kontinuierliche Erfassung und Verarbeitung wird der Auftrag von Kleb- und/oder Dichtstoffen
   ◆ als Raupe in seiner Länge und Auftragsmenge kontrolliert und geregelt,
   ◆ in der Flächenbeschichtung, beispielsweise mit Düsenkontakt oder im Sprühverfahren, in seiner Menge, bezogen auf die Auftragsfläche, überwacht und geregelt und bei variablen Geschwindigkeiten des Trägermaterials (11) konstant gehalten oder der Geschwindigkeit angepasst aufgetragen werden.

Die erfindungsgemäße Vorrichtung kann zur Bestimmung der Dichte und Messung der aufgetragenen Menge von Kleb- und/oder Dichtstoffen eingesetzt werden. Die Genauigkeit der Messung nimmt dabei mit steigender Auftragsmenge zu.

In einer bevorzugten Ausführungsform wird die erfindungsgemäße Vorrichtung zur Bestimmung der Dichte von Kleb- und/oder Dichtstoffen, die bei den entsprechenden Verarbeitungstemperaturen eine Viskosität von 20 mPas bis 100 000 mPas besitzen, gemessen nach ASTM D 3236, verwendet. Hierzu wird eine frei wählbare Menge des Kleb- und/oder Dichtstoffes am Auftragskopf (3) aufgefangen und das Gewicht des aufgefangenen Kleb- und/oder Dichtstoffes bestimmt.

Der Volumenstromsensor (5), der mit der Kontroll-Einheit (6) mit dazugehörendem Datenverarbeitungsprogramm über eine Impuls-Übertragungsleitung (8) verbunden ist, liefert eine zur abgenommenen Menge des Kleb- und/oder Dichtstoffes gehörige Impulszahl. Da pro Impuls eine definiertes Volumen des Kleb- und/oder Dichtstoffes gefördert wird, kann mit Hilfe des zur Kontroll-Einheit (6) gehörenden Datenverarbeitungsprogramms die Dichte errechnet und beispielsweise auf dem Display eines Computers angezeigt werden. Fertigungsbedingte Toleranzen der Meßwerkzahnräder und dadurch bedingte Abweichungen vom theoretisch erfassten Teilvolumen werden berücksichtigt und fließen als Toleranzfaktor in die Auswertung mit ein.

In einer weiteren bevorzugten Ausführungsform wird die erfindungsgemäße Vorrichtung zur Bestimmung der aufgetragenen Menge Kleb- und/oder Dichtstoffe, die bei der entsprechenden Verarbeitungstemperatur eine Viskosität von 20 mPas bis 100 000 mPas besitzen, gemessen nach ASTM D 3236, verwendet. Ist die Dichte des aufzutragenden Kleb- und/oder Dichtstoffes bekannt oder wurde sie beispielsweise durch oben beschriebene Methode bestimmt, kann zusammen mit der vom Volumenstromsensor (5) über die Impuls-Übertragungsleitung (8) an die Kontroll-Einheit (6) gelieferte Impulszahl mit Hilfe eines zur Kontroll-Einheit (6) gehörenden Datenverarbeitungsprogramms das Gewicht des geförderten Kleb- und/oder Dichtstoffes errechnet und beispielsweise auf dem Display eines Computers angezeigt werden. Fertigungsbedingte Toleranzen der Meßwerkzahnräder und dadurch bedingte Abweichungen vom theoretisch erfassten Teilvolumen werden berücksichtigt und fließen als Toleranzfaktor in die Auswertung mit ein.

Idealerweise bleibt nach der so erfolgten Kalibrierung die Dichte des Kleb- und/oder Dichtstoffes konstant.

Somit wird insbesondere eine Kleinstmengenmessung und -dosierung des aufgetragenen bzw. aufzutragenen Kleb- und/oder Dichtstoffes ermöglicht.

Unter Kleinstmengen werden, wie bereits an anderer Stelle ausgeführt, Mengen von 10 mg bis 100 mg, bezogen auf eine Dichte von 1 g/ml, verstanden.

Insbesondere bei der Flächenbeschichtung von Trägermaterialien (11) mit Kleb- und/oder Dichtstoffen, beispielsweise bei der Beschichtung von nonwovens mit Klebstoffen, hat das erfindungsgemäße Verfahren gegenüber den bekannten Verfahren den Vorteil, daß das Gewicht des aufgetragenen Kleb- und/oder Dichtstoffes direkt im In-line Prozeß erfaßt wird und nicht beispielsweise Off-line durch stichprobenartige Flächengewichtsbestimmung von Trägermaterialien (11) bestimmt werden muß.

Zur zusätzlichen Kontrolle, ob neben der exakten Auftragsmenge auch eine gleichmäßige Verteilung des Kleb- und/oder Dichtstoffes auf dem Trägermaterial stattgefunden hat, wird gegebenenfalls eine entsprechende und bekannte Meßvorrichtung mit Infrarotsensoren im Verfahren verwendet.

Das erfindungsgemäße Verfahren zum Auftragen von Kleb- und/oder Dichtstoffen bietet gegenüber den herkömmlichen Verfahren eine absolute Meßgenauigkeit von +/- 0,5 % der Auftragsmenge. Unter herkömmlichen Verfahren sind insbesondere Verfahren zu verstehen, bei denen der Kleb- und/oder Dichtstoff auf Trägermaterialien (11), die in Form von Zuschnitten aus den verschiedensten Werkstoffen vorliegen, aufgetragen wird. Zur Kontrolle der Auftragsmenge wird beispielsweise das Gewicht von bis zu 100 Trägermaterialien (11) vor und nach dem Auftrag des Kleb- und/oder Dichtstoffes bestimmt, statistisch ausgewertet und entsprechend der Auswertung die Auftragsvorrichtung kalibriert. Häufig ist das Gewicht des aufgetragenen Kleb- und/oder Dichtstoffes relativ zum Gewicht des Trägermaterials (11) gering, was in einer höheren Messungenauigkeit der Gewichtsbestimmung resultiert. Weitere Faktoren, beispielsweise Feuchtigkeitsabgabe aus dem Trägermaterial oder dem Kleb- und/oder Dichtstoff nach Auftrag auf das Trägermaterial (11) und vor der Gewichtsbestimmung erhöhen die Streubreite der Messung.

Die Genauigkeit, mit der Kleb- und/oder Dichtstoffe erstmalig im produktionstechnischem Maßstab konstant aufgetragen und dosiert werden sowie mit der die Auftragsmenge bestimmt werden kann, ermöglicht die Verwendung der erfindungsgemäßen Vorrichtung nicht nur als permanent installierte Vorrichtung in bereits bestehende Verfahren zum Auftragen von Kleb- und/oder Dichtstoffen, sondern auch als zeitlich begrenzt installierte Vorrichtung in bestehende Anlagen, beispielsweise als Meß- und Regeleinrichtung zur Überprüfung neuer Kleb- und/oder Dichtstoffe.

Es ist hervorzuheben, dass alle Messverfahren nach dem Stand der Technik, welche den tatsächlichen Auftrag auf dem Trägermaterial in laufender Produktion messen, nur bedingt zur Bestimmung der Auftragsmenge eingesetzt werden können. Eine Flächenbeschichtung ist sicherlich noch reproduzierbar zu messen, bei Punkt- und Raupenauftrag ist eine sichere Messung bezüglich der Länge und Position möglich, nicht aber die Bestimmung der mg-genauen Menge. Bei einem Sprühauftrag mit z. B. niedrigviskoser Flüssigkeit auf z. B. gleichfarbigem Vlies ist eine sichere Messung nicht mehr möglich. Hinzu kommt, dass Messsysteme für den tatsächlichen Auftrag immer auf die Anwendung gezielt auszulegen sind und diese durch äußere Störeinflüsse, wie z. B. Veränderung der Umgebungstemperatur, Farbveränderungen, Feuchtegehalt, usw. fehlerhafte Messergebnisse liefern. Das erfindungsgemäße Messverfahren ist universell einsetzbar und reagiert nicht auf die beschriebenen Störeinflüsse. Die Messung der Durchstrommenge garantiert die mg-genaue Auftragsmenge. Dies ist eine Voraussetzung für eine genaue Mengenregelung.

Die erfindungsgemäße Vorrichtung sowie deren Verwendung in Verfahren zum Auftragen von Kleb- und/oder Dichtstoffen trägt im hohen Masse zur Verbesserung der Qualität bei. Durch das kontinuierliche Messen und Regeln der geförderten Kleb- und/oder Dichtstoffmenge wird weniger Ausschuß produziert. Die in der Praxis üblichen 10 - 30 Gew.-% Zusatzreserve zur theoretisch benötigten Kleb- und/oder Dichtstoff-Auftragsmenge entfällt oder kann zumindest drastisch reduziert werden. Die mit der Überdosierung von Kleb- und/oder Dichtstoff verbundenen Nachteile, wie beispielsweise das Festkleben der beschichteten Trägermaterialien (11) auf den Transporteinrichtungen (12) oder die Verschmutzung von Anlagen durch Kleb- und/oder Dichtstoffreste, entfallen somit ebenfalls.

Das erfindungsgemäße Verfahren unter Verwendung der erfindungsgemäßen Vorrichtung eignet sich insbesondere zu Auftragen von Klebstoffen, bevorzugt Schmelzklebstoffen, auf Trägermaterialien (11) für Medikalartikel und Hygieneartikel.

Es ist denkbar, dass die erfindungsgemäße Vorrichtung sowie deren Verwendung in Verfahren zum Auftragen und Auftragsmessung von Kleb- und/oder Dichtstoffen auch zum Auftragen und Auftragsmessung nieder- und hochviskose Flüssigkeiten aller Art verwendet werden kann, insbesondere Flüssigkeiten, deren Viskosität bei entsprechenden Verarbeitungstemperaturen 20 mPas bis 100 000 mPas beträgt, gemessen nach ASTM D 3236.

Flüssigkeiten, die eine solche Voraussetzung erfüllen, werden beispielsweise in der Nahrungsmittel-, Kosmetikindustrie oder chemischen Industrie verarbeitet und sind beispielsweise Pasten oder Farben.

Die erfindungsgemäße Vorrichtung zum geregelten Auftrag von Kleb- und/oder Dichtstoffen auf Trägermaterialien wird nachfolgend an Hand der zeichnerischen Darstellung eines Ausführungsbeispiels näher erläutert.
Fig. 1 zeigt:
   Perspektivisch die neuartige Vorrichtung mit einem unmittelbar vor den Auftragsdüsen angeordneten Volumenstromsensor und der Kontroll-Einheit als Meß- und Regeleinheit.
Fig. 2 zeigt:
   Im Schnitt ein konkretes Ausführungsbeispiel des Gehäuses zur Aufnahme des Volumenstromsensors und
Fig. 3 zeigt:
   Perspektivisch einen Volumenstromsensor bekannter Art.
Fig. 4 zeigt: Eine Figur zur Erläuterung des Messprinzips bei intermittierendem Leimraupenauftrag
Fig. 5 zeigt: Eine Figur zur Erläuterung des Messprinzips bei kontinuierlichem Flächenauftrag

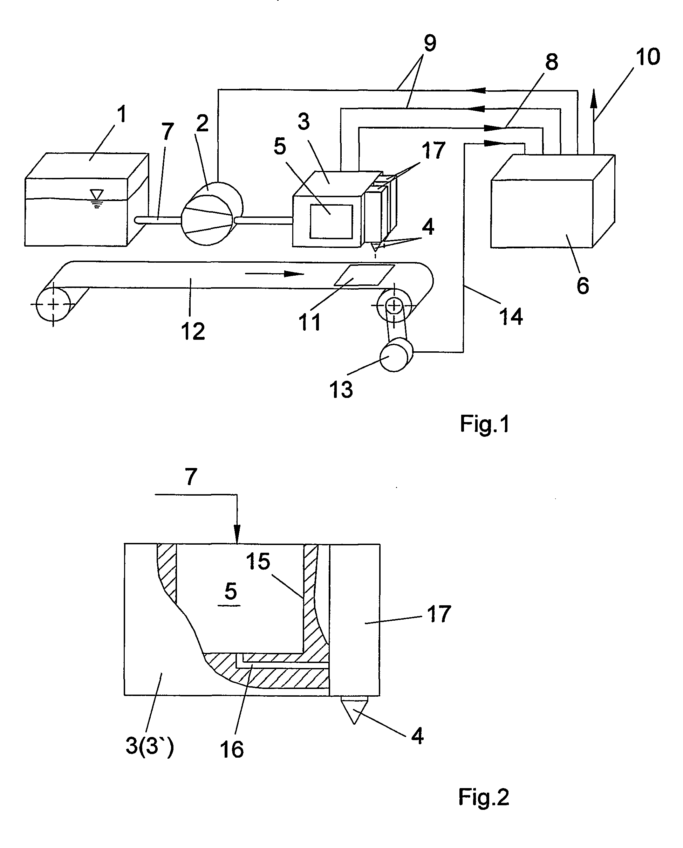

Die in Fig. 1 dargestellte Vorrichtung zum geregelten Auftragen von Kleb- und/ oder Dichtstoffen auf Trägermaterialien besteht aus
a) Vorratsbehälter für einen Kleb- und/oder Dichtstoff (1),
b) Förderpumpe (2),
c) Auftragskopf (3) mit mindestens einer Auftragsdüse (4),
d) Volumenstromsensor (5),
e) Kontroll-Einheit (6) mit dazugehörendem Datenverarbeitungsprogramm,
wobei der Vorratsbehälter (1), die Förderpumpe (2) und der Auftragskopf (3) mit mindestens einer Auftragsdüse (4) durch ein den Kleb- und/oder Dichtstoff führendes Leitungssystem (7) miteinander verbunden sind, der Volumenstromsensor (5) und die Kontroll-Einheit (6) mit dazugehörendem Datenverarbeitungsprogramm mit einer Impuls-Übertragungsleitung (8) miteinander verbunden sind, die Kontroll-Einheit (6) mit dazugehörendem Datenverarbeitungsprogramm, die Förderpumpe (2) und der Auftragskopf (3) über Steuerleitungen (9) miteinander verbunden sind und die Kontroll-Einheit (6) mit dazugehörendem Datenverarbeitungsprogramm über einen Ausgang (10) zur externen Verarbeitung von Störungsmeldungen verfügt.

Der Volumenstromsensor (5) ist zwischen der Förderpumpe (2) und den in diesem Beispiel beiden Auftragsdüsen (4) angeordnet.

Der Volumenstromsensor (5) dient, wie vorerwähnt, zur Volumenstromkontrolle des von der Förderpumpe (2) aus dem Vorratsbehälter (1) zu den Auftragsdüsen (4) geförderten Dicht- und/oder Klebstoffes.

Der Volumenstromsensor (5) ist Bestandteil des Auftragkopfes (3) und, in Flußrichtung des geförderten Kleb- und/oder Dichtstoffes gesehen, unmittelbar vor den beiden Auftragsdüsen (4) angeordnet. Durch diese Anordnungsweise werden auftretende Druckschwankungen zwischen den Auftragsdüsen (4) und dem Volumenstromsensor (5) minimiert und damit die die Messgenauigkeit verbessert. Der Volumenstrom des durch den Volumenstromsensor (5) hindurchströmenden Kleb- und/oder Dichtstoffes wird in Form elektrischer Impulse erfaßt und an die Kontroll-Einheit (6) geleitet.

Wesentlich für die Vorrichtung ist nun, daß die Kontroll-Einheit (6) die elektrischen Impulse mit einem entsprechendem dazugehörendem Datenverarbeitungsprogramm verarbeitet und über einen kontinuierlichen Soll-/Ist-Abgleich In-line die Auftragsmenge des Kleb- und/oder Dichtstoffes auf das Trägermaterial (11) an den Sollwert anpasst, indem die Förderpumpe (2) angesteuert und die Leistung entsprechend erhöht oder erniedrigt wird.

In der dargestellten bevorzugten Ausführungsform ist die Kontroll-Einheit (6) über eine entsprechende Übertragungsleitung (14) mit einem Encoder (13) verbunden. Mit Hilfe des Encoders (13) ist es möglich, den Weg und die dazu benötigte Zeit von Trägermaterialien (11) zu erfassen, die an den Auftragsdüsen (4) vorbeigeführt werden. Die vom Encoder (13) gelieferten Informationen werden von der Kontroll-Einheit (6) mit dazugehörendem Datenverarbeitungsprogramm erfasst, elektronisch verarbeitet und ermöglichen eine geschwindigkeitsabhängige Regelung der Auftragsmenge sowie der Auftragslänge des Kleb- und/oder Dichtstoffes.

In Fig.1 ist weiterhin zum besseren Verständnis eine kontinuierlich oder diskontinuierlich laufenden Transporteinrichtung (12), beispielsweise ein Förderband, und ein darauf befindliches Trägermaterial (11), beispielsweise ein zu beklebender Verpackungszuschnitt, dargestellt.

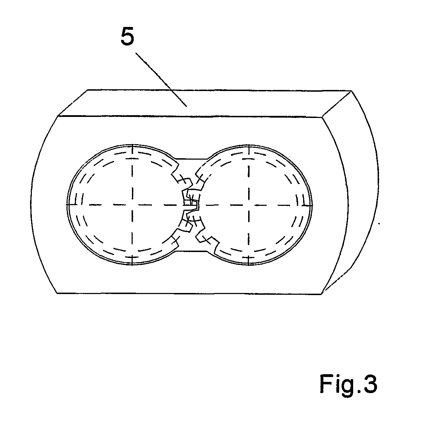

Vollständigkeitshalber ist in Fig. 3 ein handelsüblicher Volumenstromsensor (5) dargestellt, der insoweit keiner näheren Erläuterung bedarf.

In konkreter und bevorzugter Ausführungsform der Vorrichtung ist der Auftragskopf (3) in Form eines quaderförmigen Gehäuses (3') ausgebildet und mit einer der Gehäuseform des Volumenstromsensors (5) entsprechenden Ausnehmung (15) versehen, in der der Volumenstromsensor (5) eingesetzt ist, der abströmseitig über einen Kanal (16) im Gehäuse (3') mit dem außen am Gehäuse (3') angesetzten Ventilgehäuse (17) der mindestens einen Auftragsdüse (4) in Verbindung steht.

Auch das in Fig. 2 angedeutete Ventilgehäuse (17) bedarf keiner näheren Erläuterung, da sich an solchen Ventilgehäusen (17) mit ihren Auftragsdüsen (4) nichts ändert.

### Beschreibung des Meßprinzips

Zum besseren Verständnis wird das Meßprinzip anhand dreier Beispiele erläutert.
1. Intermittierender Leimraupenauftrag
2. Kontinuierlicher Flächenauftrag
3. Dosieren

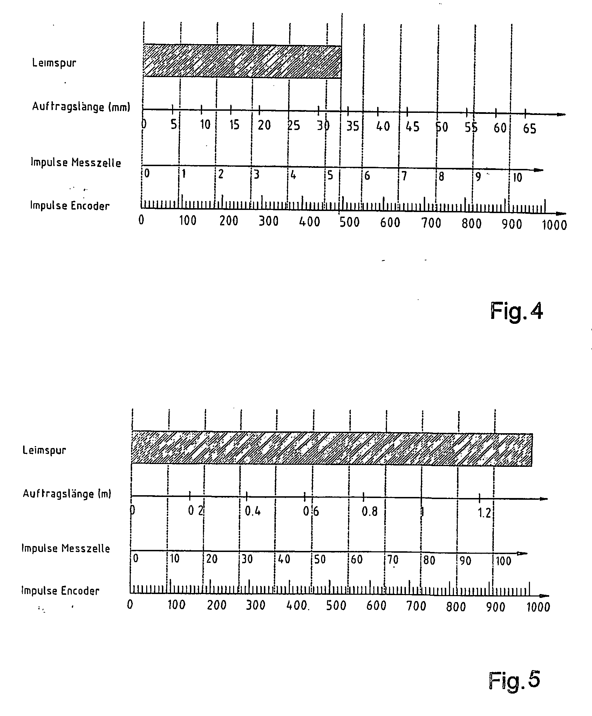

### 1. Intermittierender Leimraupenauftrag (siehe Fig. 4)

Folgende Daten liegen der Messung zugrunde:
- Auftragslänge 34 mm
- Volumenstromsensor mit Auflösung 1 Impuls = 10 mg (Dichte = 1g/ml)
- Encoder mit 1000 Impulsen/Umdrehung
- Übersetzung Encoder - Transportband: 1 Umdrehung Encoder = 69 mm Transportweg

Gemessen werden folgende Daten:
- Öffnungszeit der Leimmodule (Ventilgehäuse) = Triggerlänge Messung = 0,2 s
- Impulse Volumenstromsensor = 5
- Impulse Encoder = 490
- Verhältnis Impulse Volumenstromsensor/Encoder = konstant
- Bandgeschwindigkeit = 10,2 m/min

Über Triggerlänge und Bandgeschwindigkeit ergibt sich die Leimspurlänge von 34 mm.

Die Auftragsmenge liegt zwischen dem 5. und 6. Impulse des Volumenstromsensors. Der erfasste Anteil von 5 Impulsen beträgt 50 mg bei 455 Impulsen des Encoders. Teilt man die 5 Impulse des Volumenstromsensors durch die 455 Impulse des Encoders, so erhält man eine Impulswertigkeit von 0,010989 = 1/91.

Die Restmenge nach dem 5. Impuls errechnet sich aus: 490 - 455 = 35 Encoderimpulse;
35 Encoderimp. × 1/91 Impulswertigkeit = 0,3846 Impulse Volumenstromsensor ×
10 mg/Imp = 3,846 mg;
Es ergibt sich die Auftragsmenge mit 50 + 3,846 = 53,846 mg

### 2. Kontinuierlicher Flächenauftrag (siehe Figur 5)

Folgende Daten liegen der Messung zugrunde:
- Auftragsbreite 50 mm
- Volumenstromsensor mit Auflösung 1 Impuls = 10 mg (Dichte = 1g/ml)
- Encoder mit 1000 Impulsen/Umdrehung
- Übersetzung Encoder - Folienbahn: 1 Umdrehung Encoder = 1,375 m Folienbahn

Gemessen werden folgende Daten:
- Meßzyklus alle 50 eingehenden Impulse des Volumenstromsensors
- Impulse Encoder je Meßzyklus = 455

Mit dem 50. Impuls des Volumenstromsensors werden vom Encoder 455 Impulse gemessen.
455 Impulse Encoder = 0,625625 m Folienbahn.

Mit der Auftragsbreite von 50 mm ergibt sich eine Auftragsfläche von 0,05 m x 0,625625 m = 0,0312812 m² und mit 50 Impulse Volumenstromsensor = 500 mg = 0,5g ergibt sich das Flächenauftragsgewicht von 0,5 g/0,0312812 m² =15,984g/m². Da der Meßzyklus von den Impulsen des Volumenstromsensors ausgelöst wird, ergibt sich bei zunehmendem Auftragsgewicht eine kürzere Auftragslänge, d.h. das nächste meßbare höhere Auftragsgewicht ergibt sich mit dem 50. Impuls Volumenstromsensor und 454 Impulsen Encoder.
454 Impulse Encoder = 0,62425 m Folienbahn x 0,05 m = 0,0312125 m². Flächenauftragsgewicht = 0,5 g/0,0312125 m² = 16,0192 g/m².

Das nächste meßbare geringere Auftragsgewicht ergibt sich mit dem 50. Impuls Volumenstromsensor und 456 Impulse Encoder = 0,627 m Folienbahn x 0,05 m = 0,03135 m²_{.}
Flächenauftragsgewicht = 0,5 g/0,03135 m² = 15.9489 g/m².

Da in diesem Meßverfahren die Auflösung des Encoders für die Mengenbestimmung zugrundegelegt wird, ergibt sich in diesem Beispiel eine Meßwertgenauigkeit von +/- 0.11 %, da 455 = 100% und 1 Impuls = 0.22 % sind.

### 3. Dosieren

Das Meßsystem kann als Dosiereinheit eingesetzt werden. Die Dosiermenge bewegt sich im Raster des Volumenstromsensors, d.h. z.B. (Dichte = 1 g/ml) 10 mg + 10 mg +...+10 mg + ...

In dieser Version ist ein Encoder nicht erforderlich. Die Kontrolleinheit steuert über eine Steuerleitung das Ventilgehäuse und somit die Öffnungszeit.

Die Dosiermenge wird vorgegeben, indem die Impulszahl des Volumenstromsensors an der Kontolleinheit als Soll eingestellt wird. Zusätzlich wird eine max. Dosierzeit eingebenen. Im Dosiervorgang wird das Erreichen der Sollmenge sowie die dafür benötigte Dosierzeit überwacht. Die Dosiermenge wird immer erreicht, solange Füllgut (Klebstoff) unter Druck im Auftragssystem vorhanden ist. Änderungen in der Durchflußrate aufgrund von Druckveränderung bzw. Verstopfung in Filter od. Düsen des Ventilgehäuses wird durch Veränderung der Dosierzeit erfaßt . Die Förderpumpe (Druck) wird von der Kontrolleinheit gesteuert, sodaß die Dosier/menge innerhalb der vorgegebenen Dosierzeit fließt. Wird die Dosiermenge nicht innerhalb der max. vorgegebenen Dosierzeit erreicht, erfolgt eine Fehlermeldung.

### Bezugszeichenliste:

- (1): Vorratsbehälter für einen Kleb- und/oder Dichtstoff,
- (2): Förderpumpe,
- (3): Auftragskopf mit mindestens einer
- (4): Auftragsdüse,
- (5): Volumenstromsensor,
- (6): Kontroll-Einheit mit dazugehörendem Datenverarbeitungsprogramm,
- (7): Leitungssystem, verbindet Vorratsbehälter (1), die Förderpumpe (2) und der Auftragskopf (3) mit mindestens einer Auftragsdüse (4),
- (8): Impuls-Übertragungsleitung, verbindet Volumenstromsensor (5) und die Kontroll-Einheit (6),
- (9): Steuerleitungen (9), verbinden Kontroll-Einheit (6), Förderpumpe (2) und Auftragskopf (3),
- (10): Ausgang zur externen Verarbeitung von Störungsmeldungen (an Kontroll- Einheit (6)),
- (11): Trägermaterial,
- (12): Transporteinrichtung,
- (13): Encoder oder auch Drehwinkelgeber,
- (14): Übertragungsleitung, verbindet Encoder (13) und Kontroll-Einheit (6)
- (15): Ausnehmung in der Sonderform (3') des Auftragskopfes (3)
- (16): Kanal
- (17): Ventilgehäuse

## Patentansprüche

1. Vorrichtung zum geregelten Auftragen von Kleb- und Dichtstoffen auf Trägermaterialien (11), die die folgenden Bauteile enthält,
a) Vorratsbehälter für mindestens einen Kleb- und/oder Dichtstoff (1),
b) Förderpumpe (2),
c) Auftragskopf (3) mit mindestens einer Auftragsdüse (4),
d) Volumenstromsensor (5),
wobei der Vorratsbehälter (1), die Förderpumpe (2) und der Auftragskopf (3) mit mindestens einer Auftragsdüse (4) durch ein den Kleb- und/oder Dichtstoff führendes Leitungssystem (7) miteinander verbunden sind, wobei der Volumenstromsensor (5) in Form einer umgekehrt arbeitenden Zahnradpumpe ausgebildet und Bestandteil des Auftragkopfes (3) ist und, in Flußrichtung des geförderten Kleb- und/oder Dichtstoffes gesehen, unmittelbar vor der mindestens einen Auftragsdüse (4) angeordnet ist, **dadurch gekennzeichnet, dass** eine
Kontroll-Einheit (6) mit dazugehörendem Datenverarbeitungsprogramm vorhanden ist, und
der Volumenstromsensor (5) und die Kontroll-Einheit (6) mit dazugehörendem Datenverarbeitungsprogramm mit einer Impuls-Übertragungsleitung (8) miteinander verbunden sind und
die Kontroll-Einheit (6) mit dazugehörendem Datenverarbeitungsprogramm, die Förderpumpe (2) und der Auftragskopf (3) über Steuerleitungen (9) miteinander verbunden sind wobei die Kontrolleinheit über einen kontinuierlichen Soll/Ist-Abgleich in-line die Auftragsmenge an den Sollwert anpasst.

2. Vorrichtung nach mindestens Anspruch 1, **dadurch gekennzeichnet, dass** der Auftragskopf (3) in Form eines quaderförmigen Gehäuses (3') ausgebildet und mit einer der Gehäuseform des Volumenstromsensors (5) entsprechenden Ausnehmung (15) versehen ist, in der der Volumenstromsensor (5) eingesetzt ist, der abströmseitig über einen Kanal (16) im Gehäuse (3') mit dem außen am Gehäuse (3') angesetzten Ventilgehäuse (17) der mindestens einen Auftragsdüse (4) in Verbindung steht.

3. Vorrichtung nach mindestens einem der Ansprüche 1 bis 2, **dadurch gekennzeichnet, daß** der Volumenstromsensor (5) in handelsübliche Auftragsköpfe (3) integriert ist, welche zur Erzeugung von Punkten, Raupen oder zur Flächenbeschichtung mit Kleb- und/oder Dichtstoffen verwendet werden.

4. Vorrichtung nach Anspruch 1, **dadurch gekennzeichnet, dass** die Kontroll-Einheit (6) mit dazugehörendem Datenverarbeitungsprogramm über eine entsprechende Übertragungsleitung (14) mit einem Encoder (13) einer Transportvorrichtung verbunden ist.

5. Vorrichtung nach Anspruch 1, **dadurch gekennzeichnet, dass** die Kontroll-Einheit (6) mit dazugehörendem Datenverarbeitungsprogramm über einen Ausgang (10) zur externen Verarbeitung von Störungsmeldungen verfügt.

6. Vorrichtung nach mindestens einem der Ansprüche 1 bis 5, **dadurch gekennzeichnet, daß** sie
in bereits bestehenden Verfahrensanlagen zum Auftragen von Kleb- und/oder Dichtstoffen installiert werden kann.

7. Verfahren zum geregelten Auftragen von Kleb- und/oder Dichtstoffen auf Trägermaterialien (11) unter Verwendung einer Vorrichtung nach den Ansprüchen 1 bis 6, **dadurch gekennzeichnet, dass**
a) der Volumenstrom der geförderten Kleb- und/oder Dichtstoffes mittels des Volumenstromsensors (5) unmittelbar vor der Auftragsdüse gemessen wird,
b) die vom Volumenstromsensor (5) erfassten elektrischen Impulse an die Kontroll-Einheit (6) mit dazugehörendem Datenverarbeitungsprogramm geleitet werden,
c) die Kontroll-Einheit (6) mit dazugehörendem Datenverarbeitungsprogramm über einen kontinuierlichen Soll-/Ist-Abgleich In-line die Auftragsmenge des Kleb- und/oder Dichtstoffes auf das Trägermaterial (11) an den Sollwert anpasst, indem die Förderpumpe (2) angesteuert und die Leistung entsprechend erhöht oder erniedrigt wird.

8. Verfahren nach Anspruch 7, **dadurch gekennzeichnet, dass** die Kontroll-Einheit (6) mit dazugehörendem Datenverarbeitungsprogramm die Förderung des Kleb- und/oder Dichtstoffes unterbricht, wenn eine Anpassung an den Sollwert nicht mehr möglich ist.

9. Verfahren nach Anspruch 7, **dadurch gekennzeichnet, dass** die Über- oder Unterschreitung gewählter Limitwerte von der Kontroll-Einheit (6) mit dazugehörendem Datenverarbeitungsprogramm als Störung registriert, verarbeitet und über elektronische und/oder akustische Funktionen als Störungsmeldung signalisiert wird.

10. Verfahren nach den Ansprüchen 7 bis 9, **dadurch gekennzeichnet, dass** die von einem Encoder (13) einer Transportvorrichtung gelieferten Informationen von der Kontroll-Einheit (6) mit dazugehörendem Datenverarbeitungsprogramm erfasst und elektronisch verarbeitet werden und die Auftragsmenge sowie die Auftragslänge des Kleb- und/oder Dichtstoffes in Abhängigkeit von der Geschwindigkeit regeln.

11. Verfahren nach Anspruch 7, **dadurch gekennzeichnet, dass** es sich bei den Klebstoffen um Leim, Kleister, Dispersions-Klebstoffe, Lösungsmittel-Klebstoffe, Kontakt-Klebstoffe, Reaktions-Klebstoffe, Haft-Klebstoffe, Haft-Schmelzklebstoffen und/oder Schmelzklebstoffe handelt.

12. Verfahren nach mindestens einem der Ansprüche 7 bis 11, **dadurch gekennzeichnet, dass** die Kleb- und/oder Dichtstoffe bei den entsprechenden Verarbeitungstemperaturen eine Viskosität von 20 mPas bis 100 000 mPas, gemessen nach ASTM D 3236, aufweisen.

13. Verfahren nach mindestens einem der Ansprüche 7 bis 12, **dadurch gekennzeichnet, dass** die aufzutragenden Kleb- und/oder Dichtstoffe bei der entsprechenden Verarbeitungstemperatur über eine konstante Viskosität mit einer Schwankung von +/- 50 % innerhalb von übliche Verarbeitungszeiträumen verfügen.

14. Verfahren nach mindestens einem der Ansprüche 7 bis 13, **dadurch gekennzeichnet, dass** das geregelte Auftragen der Kleb- und/oder Dichtstoffe auf Trägermaterialien (11) intermittierend oder kontinuierlich durchgeführt wird.

15. Verwendung der Vorrichtung nach den Ansprüchen 1 bis 6 zur Bestimmung der Dichte von Kleb- und/oder Dichtstoffen, die bei den entsprechenden Verarbeitungstemperaturen eine Viskosität von 20 mPas bis 100 000mPas, gemessen nach ASTM D 3236, aufweisen.

16. Verwendung der Vorrichtung nach den Ansprüchen 1 bis 6 zur Bestimmung der aufgetragenen Menge von Kleb- und/oder Dichtstoffen, die bei den entsprechenden Verarbeitungstemperaturen eine Viskosität von 20 mPas bis 100 000mPas, gemessen nach ASTM D 3236, aufweisen.

17. Verwendung der Vorrichtung nach den Ansprüchen 1 bis 6 zum geregelten Auftragen und Dosieren von Kleb- und/oder Dichtstoffen im produktionstechnischem Maßstab.

18. Verwendung der Vorrichtung nach den Ansprüchen 1 bis 6 und des Verfahrens nach den Ansprüchen 7 bis 14 zur Kleinstmengenmessung und -dosierung, wobei unter Kleinstmengen Mengen von 10 mg bis 100 mg, bezogen auf eine Dichte von 1 g/ml, verstanden werden.

19. Verwendung der Vorrichtung nach den Ansprüchen 1 bis 6 zum Auftragen von Klebstoffen auf Trägermaterialien (11) für Medikalartikel und Hygieneartikel.

## Claims

1. A device for controlled application of adhesives and sealants on support materials (11), which contains the following components,
a) a storage container for at least one adhesive and/or sealant (1),
b) a feed pump (2),
c) an application head (3) with at least one application nozzle (4),
d) a volume flow sensor (5),
wherein the storage container (1), the feed pump (2) and the application head (3) with at least one application nozzle (4), are connected to each other through a piping system (7), conducting the adhesive and/or sealant, wherein the volume flow sensor (5) is formed as a reversely operating gear pump and is part of the application head (3), and, viewed in the flow direction of the conveyed adhesive and/or solvent, is directly positioned in front of said at least one application nozzle (4), **characterized in that** a
control unit (6) with an associated data processing program is available, and
the volume flow sensor (5) and the control unit (6) with an associated data processing program are connected to each other with a pulse transmission line (8) and
the control unit (6) with an associated data processing program, the feed pump (2) and the application head (3) are connected to each other via control lines (9), wherein the control unit adapts on line the application amount to the set value via a continuous set/actual value comparison.

2. The device according to at least claim 1, **characterized in that** the application head (3) is formed as a cuboid-shaped housing (3') and is provided with a recess (15) matching the housing shape of the volume flow sensor (5), in which the volume flow sensor (5) is inserted, which is connected downstream via a channel (16) in the housing (3') with the valve housing (17) exteriorly placed on the housing (3') of the at least one application nozzle (4).

3. The device according to at least one of claims 1 to 2, **characterized in that** the volume flow sensor (5) is integrated into commercially available application heads (3), which are used for producing spots, beads or for coating surfaces with adhesives and/or sealants.

4. The device according to claim 1, **characterized in that** the control unit (6) with an associated data processing program is connected to an encoder (13) of a transport device over an adequate transmission line (14).

5. The device according to claim 1, **characterized in that** the control unit (6) with an associated data processing program has an output (10) available for external processing of malfunction messages.

6. The device according to at least one of claims 1 to 5, **characterized in that** it may be installed in already existing processing plants for applying adhesives and/or sealants.

7. A method for controlled application of adhesives and/or sealants on support materials (11) by the use of a device according to claims 1 to 6, **characterized in that**
a) the volume flow of the conveyed adhesive and/or sealant is measured by means of the volume flow sensor (5) directly in front of the application nozzle,
b) the electric pulses acquired from the volume flow sensor (5) are transmitted to the control unit (6) with an associated data processing program,
c) the control unit (6) with an associated data processing program via a continuous set/actual value comparison adapts in-line the application amount of the adhesive and/or sealant onto the support material (11) to the set value, by driving the feed pump (2) and correspondingly increasing or decreasing the power.

8. The method according to claim 7, **characterized in that** the control unit (6) with an associated data processing program interrupts the conveyance of the adhesive and/or sealant if an adaptation to the set value is no longer possible.

9. The method according to claim 7, **characterized in that** the exceeding or falling below of selected limiting values is recorded as a malfunction by the control unit (6) with an associated data processing program, processed and indicated as a malfunction message via electronic or acoustic functions.

10. The method according to claims 7 to 9, **characterized in that** the information delivered from an encoder (13) of a transport device is acquired and electronically processed by the control unit (6) with an associated data processing program and the application amount as well as the application length of the adhesive and/or sealant is adjusted depending on the speed.

11. The method according to claim 7, **characterized in that** as for the adhesives these are glue, paste, dispersion adhesives, solvent adhesives, contact adhesives, reaction adhesives, pressure-sensitive adhesives, hot melt pressure-sensitive adhesives, and/or hot melt adhesives.

12. The method according to at least one of claims 7 to 11, **characterized in that** the adhesives and/or sealants have a viscosity from 20 mPa.s to 100,000 mPa.s, as measured according to ASTM D 3236, at the corresponding processing temperatures.

13. The method according to at least one of claims 7 to 12, **characterized in that** the adhesives and/or sealants to be applied, at the corresponding processing temperature, have a constant viscosity with a variation of ± 50% within customary processing time periods.

14. The method according to at least one of claims 7 to 13, **characterized in that** the controlled application of the adhesives and/or sealants on support materials (11) is carried out intermittently or continuously.

15. The use of the device according to claims 1 to 6 for determining the density of adhesives and/or sealants, which have a viscosity from 20 mPa.s to 100,000 mPa.s, as measured according to ASTM D 3236, at the corresponding processing temperatures.

16. The use of the device according to claims 1 to 6 for determining the applied amount of adhesives and/or sealants which have a viscosity from 20 mPa.s to 100,000 mPa.s, as measured according to ASTM D 3236, at the corresponding processing temperatures.

17. The use of the device according to claims 1 to 6 for controlled application and metering of adhesives and/or sealants at an industrial production scale.

18. The use of the device according to claims 1 to 6 and of the method according to claims 7 to 14 for measuring and metering small quantities, by small quantities being meant amounts from 10 mg to 100 mg, based on a density of 1g/ml.

19. The use of the device according to claims 1 to 6 for applying adhesives on support materials (11) for medical articles and hygiene articles.

## Revendications

1. Dispositif pour l'application réglée de substances adhésives et d'étanchéité sur des matières de support (11), qui contient les composants suivants :
a) un récipient de stockage pour au moins une substance adhésive et/ou d'étanchéité (1) ;
b) une pompe d'alimentation (2) ;
c) une tête d'application (3) comprenant au moins une buse d'application (4) ;
d) un capteur du débit volumétrique (5) ;
le récipient de réserve (1), la pompe d'alimentation (2) et la tête d'application (3) comprenant au moins une buse d'application (4) étant reliés l'un à l'autre via un système de conduction (7) guidant la substance adhésive et/ou d'étanchéité, le capteur du débit volumétrique (5) étant réalisé sous la forme d'une pompe à roue dentée travaillant à l'inverse et représentant un élément constitutif de la tête d'application (3), tout en étant disposé, vu dans la direction d'écoulement de la substance adhésive et/ou d'étanchéité acheminée, directement devant ladite au moins une buse d'application (4), **caractérisé en ce qu'**est présente une unité de commande (6) comprenant un programme de traitement de données qui lui est associé, et **en ce que** le capteur du débit volumétrique (5) et l'unité de commande (6) comprenant un programme de traitement de données qui lui est associé sont reliés l'un à l'autre avec une ligne de transmission d'impulsions (8), et **en ce que** l'unité de commande (6) avec le programme de traitement de données qui lui est associé, la pompe d'alimentation (2) et la tête d'application (3) sont reliés l'un à l'autre via des lignes de commande (9), l'unité de commande adaptant en ligne via une synchronisation en continu entre la valeur de consigne et la valeur réelle, la quantité d'application à la valeur de consigne.

2. Dispositif selon au moins la revendication 1, **caractérisé en ce que** la tête d'application (3) est réalisée sous la forme d'un logement parallélépipédique (3') et est munie d'un évidement dont la forme correspond à celle du logement du capteur du débit volumétrique, dans lequel vient se disposer le capteur du débit volumétrique (5) qui est mis en liaison, côté écoulement, via un canal (16) dans le logement (3'), avec le corps de vanne (17) de ladite au moins une buse d'application (4) appliqué contre l'extérieur du logement (3').

3. Dispositif selon au moins une des revendications 1 à 2, **caractérisé en ce que** le capteur du débit volumétrique (5) est intégré dans des têtes d'application (3) disponibles dans le commerce que l'on utilise pour l'obtention de points, de cordons ou bien pour l'enduction de surfaces avec des substances adhésives et/ou d'étanchéité.

4. Dispositif selon la revendication 1, **caractérisé en ce que** l'unité de commande (6) avec le programme de traitement de données qui lui est associé est reliée via une ligne de transmission correspondant (14) à un encodeur (13) d'un dispositif de transport.

5. Dispositif selon la revendication 1, **caractérisé en ce que** l'unité de commande (6) avec le programme de traitement de données qui lui est associé dispose d'une sortie (10) pour le traitement externe de signalements de perturbation.

6. Dispositif selon au moins une des revendications 1 à 5, **caractérisé en ce qu'**on peut le monter dans des installations de traitement déjà existantes pour l'application de substances adhésives et/ou d'étanchéité.

7. Procédé pour l'application réglée de substances adhésives et/ou d'étanchéité sur des matières de support (11) en utilisant un dispositif selon les revendications 1 à 6, **caractérisé en ce que**
a) le débit volumétrique de la substance adhésive et/ou d'étanchéité acheminée est mesuré au moyen du capteur du débit volumétrique (5) directement avant la buse d'application ;
b) les impulsions électriques enregistrées par le capteur du débit volumétrique (5) sont amenées à l'unité de commande (6) avec un programme de traitement de données qui lui est associé ;
c) l'unité de commande (6) avec le programme de traitement de données qui lui est associé adapte en ligne, via une synchronisation en continu entre la valeur de consigne et la valeur réelle, la quantité d'application de la substance adhésive et/ou d'étanchéité sur la matière de support (11) à la valeur de consigne, en réglant la pompe d'alimentation (2) et en augmentant ou en diminuant la puissance de manière correspondante.

8. Procédé selon la revendication 7, **caractérisé en ce que** l'unité de commande (6) avec le programme de traitement de données qui lui est associé, interrompt l'alimentation de la substance adhésive et/ou d'étanchéité lorsqu'une adaptation à la valeur de consigne n'est plus possible.

9. Procédé selon la revendication 7, **caractérisé en ce que** le dépassement vers le haut ou vers le bas de valeurs limites sélectionnées est enregistré comme perturbation, est traité et est signalé via des fonctions électroniques et/ou acoustiques sous la forme d'un signalement de perturbation par l'unité de commande (6) avec le programme de traitement de données qui lui est associé.

10. Procédé selon les revendications 7 à 9, **caractérisé en ce que** les informations fournies par un encodeur (13) d'un dispositif de transport sont collectées et traitées électroniquement par l'unité de commande (6) avec le programme de traitement de données qui lui est associé et règlent la quantité d'application ainsi que la longueur d'application de la substance adhésive et/ou d'étanchéité en fonction de la vitesse.

11. Procédé selon la revendication 7, **caractérisé en ce que**, en ce qui concerne les substances adhésives, il s'agit de colle, de colle d'amidon, de substances adhésives de dispersion, d'adhésifs contenant un ou plusieurs solvants, d'adhésifs de contact, d'adhésifs réactionnels, d'adhésifs sensibles à la pression, d'adhésifs thermofusibles sensibles à la pression et/ou d'adhésifs thermofusibles.

12. Procédé selon au moins une des revendications 7 à 11, **caractérisé en ce que** les substances adhésives et/ou d'étanchéité présentent, aux températures de traitement correspondantes, une viscosité de 20 mPas à 100.000 mPas, mesurée conformément à la norme ASTM D 3236.

13. Procédé selon au moins une des revendications 7 à 12, **caractérisé en ce que** les substances adhésives et/ou d'étanchéité à appliquer disposent, à la température de traitement correspondante, d'une viscosité constante avec une fluctuation de ± 50 % au sein de laps de temps de traitement habituels.

14. Procédé selon au moins une des revendications 7 à 13, **caractérisé en ce que** l'application réglée des substances adhésives et/ou d'étanchéité sur des matières de support (11) est mise en oeuvre de manière intermittente ou en continu.

15. Utilisation du dispositif selon les revendications 1 à 6, pour la détermination de la densité des substances adhésives et/ou d'étanchéité qui présentent aux températures de traitement correspondantes, une viscosité de 20 mPas à 100.000 mPas, mesurée conformément à la norme ASTM D 3236.

16. Utilisation du dispositif selon les revendications 1 à 6, pour la détermination de la quantité appliquée de substances adhésives et/ou d'étanchéité qui présentent aux températures de traitement correspondantes, une viscosité de 20 mPas à 100.000 mPas, mesurée conformément à la norme ASTM D 3236.

17. Utilisation du dispositif selon les revendications 1 à 6, pour l'application réglée et le dosage de substances adhésives et/ou d'étanchéité à l'échelle industrielle.

18. Utilisation du dispositif selon les revendications 1 à 6 et mise en oeuvre du procédé selon les revendications 7 à 14, pour le dosage et la mesure de faibles quantités, lesdites faibles quantités correspondant à des quantités de 10 mg à 100 mg, rapportés à une densité de 1 g/ml.

19. Utilisation du dispositif selon les revendications 1 à 6, pour l'application d'adhésifs sur des matières de support (11) destinées à des articles médicaux et à des articles hygiéniques.
